# EUROPÄISCHE PATENTSCHRIFT

(11) **EP 3 169 766 B1**
(45) Veröffentlichungstag und Bekanntmachung des Hinweises auf die Patenterteilung: **13.06.2018**
(21) Anmeldenummer: 15728773.1
(22) Anmeldetag: 22.05.2015
(51) Int. Cl.: C12M 1/12, C12M 1/24, C12M 1/00

(54) **LABORBEHÄLTER, INSBESONDERE ZELLKULTURBEHÄLTER, MIT EINER IN DAS BEHÄLTERVOLUMEN HINEIN VERLAUFENDEN GAS-AUSGLEICHSLEITUNG**
LABORATORY CONTAINER, IN PARTICULAR CELL CULTURE CONTAINER, COMPRISING A GAS BALANCING LINE WHICH RUNS INTO THE CONTAINER VOLUME
RÉCIPIENT DE LABORATOIRE, EN PARTICULIER RÉCIPIENT DE CULTURE CELLULAIRE, POURVU D'UN CONDUIT DE COMPENSATION DE GAZ S'ÉTENDANT DANS L'ESPACE INTÉRIEUR DU RÉCIPIENT

(30) Priorität: 18.07.2014 DE 102014214077
(43) Veröffentlichungstag der Anmeldung: 24.05.2017
(73) Patentinhaber: Hamilton Bonaduz AG, 7402 Bonaduz (CH)
(72) Erfinder: JÄGER, Thomas, 79865 Grafenhausen (DE); SEILER, Tobias, CH-7017 Flims (CH); ETZOLD, Carsten, 8634 Hombrechtikon (CH)
(74) Vertreter: RLTG
(86) Internationale Anmeldenummer: PCT/EP2015/061412
(87) Internationale Veröffentlichungsnummer: WO 2016/008628

(56) Entgegenhaltungen:
- WO-A1-2014/044612
- WO-A2-2008/112845
- DE-T2- 69 534 132

## Beschreibung

Die vorliegende Erfindung betrifft einen Laborbehälter, insbesondere Zellkulturbehälter, mit einem von einem Behälterkörper umgebenen Behältervolumen, das durch eine Behälteröffnung des Behälterkörpers von außen zugänglich ist, wobei der Behälter eine mit dem Behältervolumen kommunizierende Fluidleitung mit einer Ventilanordnung aufweist, wobei die Fluidleitung zum Durchleiten von Fluid ausgebildet ist, um Fluid aus dem Behältervolumen auszuleiten oder/und von außen in dieses einzuleiten, wobei die Fluidleitung durch die Ventilanordnung wahlweise zur Fluiddurchleitung freigebbar oder sperrbar ist, und wobei der Behälter weiter eine Gas-Ausgleichsöffnung aufweist, durch welche Gas im Gegenstrom zu einem etwaigen Fluidstrom in der Fluidleitung von außen in das Behältervolumen einleitbar oder aus diesem ausleitbar ist.

Mit einem solchen Laborbehälter ist es möglich, durch die Fluidleitung hindurch Fluid von außen in den Behälter hinein einzuleiten oder aus diesem nach außen auszuleiten. Da eine Veränderung der in dem üblicherweise abgeschlossenen Behältervolumen vorhandenen Fluidmenge ohne weitere Maßnahmen auch zu einer Veränderung des in dem Behältervolumen herrschenden Drucks führen würde, weist der Behälter die genannte Gas-Ausgleichsöffnung auf, um durch eine Gasströmung im Gegenstrom zu dem zuvor genannten Fluidstrom in der Fluidleistung einen Druckausgleich im Behältervolumen herbeizuführen.

Dann, wenn Fluid, insbesondere Flüssigkeit, in das Behältervolumen eingeleitet wird, kann Gas über die Gas-Ausgleichsöffnung aus dem Behälter hinausströmen. Ebenso kann dann, wenn Fluid, insbesondere Flüssigkeit, aus dem Behältervolumen ausgeleitet wird, Gas über die Gas-Ausgleichsöffnung in das Behältervolumen hineinströmen.

Aber auch abseits eines (Teil-)Austausches von Behälterinhalten kann die Gas-Ausgleichsöffnung während der Dauer der Benutzung des Laborbehälters, etwa während dessen Aufbewahrung in einem Klimaschrank und dergleichen, diffusionsgetrieben für einen kontinuierlichen Gasaustausch zwischen dem Gas im Behältervolumen und der den Laborbehälter außen umgebenden Atmosphäre sorgen.

Nachteilig an der Bereitstellung nur einer Gas-Ausgleichsöffnung ist, dass diese insbesondere dann, wenn sich wenigstens vorübergehend eine Flüssigkeit in dem Behälter befindet, durch Flüssigkeitströpfchen und dergleichen teilweise oder vollständig verschlossen werden kann, wodurch ein Gasaustausch erschwert oder sogar verhindert werden kann. Manche Flüssigkeiten, wie etwa Nährflüssigkeiten, weisen darin gelöst Substanzen auf, die beim Abdampfen flüssiger Bestandteile als Festkörperpartikel zurückbleiben können. In diesem Falle kann eine Gas-Ausgleichsöffnung durch Trocknen von daran unerwünschterweise anhaftender Flüssigkeit sogar dauerhaft verschlossen werden.

Da gerade Zellkulturbehälter als bevorzugt betrachtete Laborbehälter häufig nach Einbringen einer Nährlösung verschwenkt werden, um Innenwandungen des Zellkulturbehälters möglichst umfangreich mit Nährlösung zu benetzen, ist die Wahrscheinlichkeit erheblich, dass im Behältervolumen vorhandene Flüssigkeit auch an die Gas-Ausgleichsöffnung gelangt.

Aus der DE 695 34 132 T2 ist eine Zellkulturflasche mit mehreren Kammern bekannt. Der bekannte unterteilte Gewebekulturkolben ist durch eine Membran in ein Basalmediumabteil und ein Zellkulturabteil unterteilt. Im Zellkulturabteil befindet sich ein Kulturmedium, das Zellen oder Gewebe enthält. In dem Basalmediumabteil befindet sich ein Basalmedium. Auf das Zellkulturabteil kann durch eine Zellkultur-Zugangsöffnung zugegriffen werden, die von einem Deckel verschlossen ist. Am anderen Ende des bekannten Gewebekulturkolbens kann auf das Basalmediumabteil durch eine Basalmedium-Zugangsöffnung zugegriffen werden, die ebenfalls von einem Deckel verschlossen ist.

Bei der Befüllung des aus der DE 695 34 132 T2 bekannten Gewebekulturkolbens mit entweder Zellkultursuspension oder mit Basalmedium wird der Deckel der jeweiligen Zugangsöffnung von der Zugangsöffnung abgenommen und das jeweilige flüssige Medium durch die zugeordnete Zugangsöffnung in den Zellkulturkolben eingefüllt. Dabei kann Gas durch dieselbe Zugangsöffnung aus dem Gewebekulturkolben gegenstromig entweichen.

Die DE 695 34 132 T2 offenbart weiterhin, dass zur Vermeidung von thermisch bedingten Gas-Überdrücken im bekannten Gewebekulturkolben die Deckel der beiden Zugangsöffnungen für Zellkultur und Basalmedium gelockert werden können, damit sich erwärmendes und folglich ausdehnendes Gas aus dem Gewebekulturkolben durch die jeweilige Zugangsöffnung entlang des zwischen Deckel und Zugangsöffnungswandung gebildeten Spalts entweichen kann.

Der bekannte Gewebekulturkolben weist daher keine in den von den jeweiligen Zugangsöffnungen mit daran anschließendem Behälterhals gebildeten Fluidleitungen vorgesehene Ventilanordnungen auf, welche die zugeordnete Fluidleitung zur Durchströmung mit Fluid freigeben oder sperren.

Aus der WO 2014/044612 A1 ist weiter ein Einweg-Flaschenreaktortank bekannt, dessen Behältervolumen durch einen Flaschenhals zugänglich ist, welcher von einem Stopfen verschlossen ist. Der Stopfen ist von mehreren Leitungen durchdrungen, nämlich von einer Gaszufuhrleitung und einer gesondert davon ausgebildeten Gasabfuhrleitung, einer Mediumzufuhrleitung, einer Harvest-Leitung zur Entnahme von Zellkulturen und einer nicht näher erläuterten und als "Stellmittel" bezeichneten Vorrichtung.

Eine Ventilanordnung in einer Fluidleitung offenbart die WO 2014/044612 A1 nicht. Alle wenigstens der Bezeichnung nach als gasführende Leitungen offenbarten Leitungen in der WO 2014/044612 A1 sind lediglich grobschematisch als geradlinige Rohrleitungen dargestellt und enden unmittelbar nach dem Durchgang durch den den Behälterhals verschließenden Stopfen.

Es ist daher Aufgabe der vorliegenden Erfindung, den eingangs genannten Laborbehälter derart weiterzuentwickeln, dass ein Gasaustausch über die Gas-Ausgleichsöffnung bei ansonsten gleicher Funktionalität mit größerer Sicherheit als bisher über die Gebrauchsdauer des Laborbehälters hinweg erzielt wird.

Diese Aufgabe wird erfindungsgemäß gelöst durch einen Laborbehälter mit allen Merkmalen des Anspruchs 1. Die Gas-Ausgleichsöffnung ist dabei an einer Gas-Ausgleichsleitung vorgesehen, welche sich längs einer virtuellen Kanalverlaufsachse erstreckt und einen von einer Kanalwandung umgebenen Leitungskanal aufweist, der ausgehend von der Gas-Ausgleichsöffnung in das Behältervolumen verläuft und in dieses hinein öffnet.

Durch die sich an die Gas-Ausgleichsöffnung anschließende Gas-Ausgleichsleitung kann der Ort des Einströmens von Gas in das und des Ausströmens von Gas aus dem Behältervolumen an eine Stelle im Inneren des Behältervolumens verlegt werden, welche selbst beim Verschwenken eines teilweise mit Flüssigkeit gefüllten Laborbehälters von der Flüssigkeit im Behältervolumen nicht oder nur mit geringer Wahrscheinlichkeit erreicht wird.

Darüber hinaus kann die im Inneren des Behältervolumens gelegene Öffnung der Gas-Ausgleichsleitung mit einer verhältnismäßig großen Öffnungsfläche ausgestaltet sein, so dass diese durch einen einzigen Tropfen nicht vollständig verschließbar ist.

Selbst in dem Falle, dass einzelne Tropfen einer im Behältervolumen vorhandenen Flüssigkeit durch die im Behältervolumen gelegene Öffnung der Gas-Ausgleichsleitung in diese hinein gelangen sollten, werden diese bei ausreichender Länge der Gas-Ausgleichsleitung eher auf die Kanalwandung der Gas-Ausgleichsleitung auftreffen als auf die Gas-Ausgleichsöffnung. Daher ist es selbst dann, wenn Flüssigkeitstropfen in die Gas-Ausgleichsleitung gelangen sollten, unwahrscheinlich, dass diese bis zur Gas-Ausgleichsöffnung gelangen.

Die Gas-Ausgleichsleitung verläuft daher von der im Behältervolumen gelegenen Öffnung der Leitung wenigstens bis zur Gas-Ausgleichsöffnung.

Um das Erreichen der Gas-Ausgleichsöffnung für Flüssigkeitstropfen aus dem Behältervolumen weiter zu erschweren, kann die Gas-Ausgleichsleitung wenigstens einmal abgewinkelt oder mit gekrümmtem Verlauf ausgebildet sein.

Zur Erschwerung eines Flüssigkeitseintritts in die Gas-Ausgleichsleitung kann eine Innenwandung des Behälters als Eintrittsschutz dienen. Hierzu kann vorgesehen sein, dass die im Behältervolumen gelegene Öffnung der Gas-Ausgleichsleitung einer der Innenwandungen des Behälterkörpers nächstgelegen ist. Dies bedeutet, dass die Öffnung näher bei einer bestimmten Innenwandung liegt als bei den übrigen vorhandenen Innenwandungen des Laborbehälters.

Übliche Laborbehälter, insbesondere in Form eines Zellkulturbehälters, weisen als Wandungen zwei im Wesentlichen parallele Stirnwandungen und eine diese verbindende Mantelwandung auf. Die Stirnwandungen sind in der Regel eben, um ein Stapeln der Laborbehälter zu erleichtern.

Vorzugsweise ist dann die im Behältervolumen gelegene Öffnung der Gas-Ausgleichsleitung einer der Stirnwandungen des Behälterkörpers näher gelegen als der jeweils anderen Stirnwandung. Somit kann der Laborbehälter in an sich bekannter Weise durch Auflegen auf einer seiner Stirnflächen aufbewahrt werden. Durch Heranführen der Gas-Ausgleichsleitung an eine der Stirnwände kann somit ermöglicht werden, dass die Öffnung sich im Aufbewahrungszustand des Laborbehälters relativ weit vom Flüssigkeitsspiegel im Laborbehälter entfernt befindet.

Die im Behältervolumen gelegene Öffnung kann dabei bevorzugt zu der ihr näher gelegenen Stirnwandung hinweisen, um einen Flüssigkeitseintritt von im Behältervolumen vorhandener Flüssigkeit in die Gas-Ausgleichsleitung hinein weiter zu erschweren. Beispielsweise kann gemäß einer bevorzugten Weiterbildung der vorliegenden Erfindung ein Rand der Öffnung der Gas-Ausgleichsleitung parallel zu der ihr näher gelegenen Innenwandung, insbesondere einer Stirnwandung, gelegen sein, so dass zwischen dem Rand der Öffnung und der Innenwandung lediglich ein umlaufender Ringspalt vorhanden ist. Durch diesen kann zwar Gas ungestört in die Gas-Ausgleichsleitung einströmen oder von dieser ausströmen, Flüssigkeit gelangt jedoch nur erschwert aus dem Behältervolumen in die Gas-Ausgleichsleitung hinein.

Wie bereits angedeutet wurde, ist der Laborbehälter bevorzugt ein Zellkulturbehälter, von dessen beiden Stirnwandungen nur eine Stirnwandung auf ihrer dem Behältervolumen zugewandten Seite zur Anlage von adhärenten Zellen ausgebildet ist. Die andere Stirnwandung dagegen ist auf ihrer dem Behältervolumen zugewandten Seite ohne derartige Ausbildung.

In diesem Falle ist es bevorzugt, dass die im Behältervolumen gelegene Öffnung der Gas-Ausgleichsleitung der zur Anlage von adhärenten Zellen ausgebildeten Stirnwandung näher gelegen ist als der jeweils anderen Stirnwandung.

Erfindungsgemäß ist die Behälteröffnung an einem freien, dem Behälterkörper fernen Längsende eines vom übrigen Behälterkörper abstehenden Behälterhalses ausgebildet. Der übrige Behälterkörper ist nachfolgend auch als "Behälterhauptkörper bezeichnet, so dass der Behälterkörper Behälterhauptkörper und Behälterhals umfassen kann.

Aus Gründen vorteilhafter Bauraumausnutzung verläuft die Gas-Ausgleichsleitung wenigstens abschnittsweise durch den Behälterhals hindurch.

Laborbehälter werden in der Regel nicht bis zum Behälterhals gefüllt. Gerade Zellkulturbehälter werden in der Regel nur zu etwa einem Drittel ihres Behältervolumens gefüllt. Daher ist der Behälterhals von im Behältervolumen vorhandenen Flüssigkeit in der Regel unbelegt. Deshalb kann gemäß der vorliegenden Erfindung vorgesehen sein, dass die Gas-Ausgleichsleitung den Behälterhals durchsetzend in ein an den Behälterhals anschließendes Behälterhauptvolumen hineinreicht. Das Behältervolumen eines halsbehafteten Laborbehälters setzt sich dann zusammen aus dem vom Behälterhals umschlossenen Behälterhalsvolumen und dem vom Behälterhauptkörper umschlossenen Behälterhauptvolumen.

Um zum einen das Behälterhalsvolumen vorteilhaft zur Anordnung der Gas-Ausgleichsleitung zu benutzen und gleichzeitig die oben genannten Vorteile einer bezüglich der Innenwandungen des Laborbehälters asymmetrischen Anordnung der im Behältervolumen gelegenen Öffnung der Gas-Ausgleichsleitung zu nutzen, kann weiter vorgesehen sein, dass sich der Behälterhals längs einer virtuellen Halsachse erstreckt, dass sich ein im Behälterhals gelegener Halsleitungsabschnitt der Gas-Ausgleichsleitung parallel zur Halsachse erstreckt und dass ein in das Behälterhauptvolumen hinein verlaufender, auf den Halsleitungsabschnitt folgender Hauptvolumenleitungsabschnitt der Gas-Ausgleichsleitung relativ zum Halsleitungsabschnitt abgewinkelt oder/und gekrümmt ist. Der Halsleitungsabschnitt der Gas-Ausgleichsleitung erstreckt sich also zunächst parallel zur Halsachse. Ein auf den Halsleitungsabschnitt folgender Hauptvolumenleitungsabschnitt kann relativ zu dem Halsleitungsabschnitt unter einem Winkel oder mit einer Krümmung oder unter Zwischenanordnung eines gekrümmten Gas-Ausgleichsleitungsabschnitts vorgesehen sein. Hierdurch kann die dann im Behälterhauptvolumen gelegene Öffnung der Gas-Ausgleichsleitung wiederum nahe an eine der Innenwandungen herangeführt sein.

Um auch bereits vorhandene Laborbehälter mit der hier diskutierten vorteilhaften Gas-Ausgleichsleitung versehen zu können, kann die Ventilanordnung an einer von dem Behälterkörper gesondert ausgebildeten Einsatzanordnung vorgesehen sein, welche in die Behälteröffnung einsetzbar ist.

Aus demselben Grund einer möglichen Nachrüstung bereits vorhandener Laborbehälter kann vorgesehen sein, dass die Ventilanordnung an einer von dem Behälterkörper gesondert ausgebildeten Einsatzanordnung vorgesehen ist, welche in die Behälteröffnung einsetzbar ist. Vorzugsweise sind zur Verringerung der Anzahl an zur Realisierung der vorliegenden Erfindung benötigten Bauteile die Gas-Ausgleichsanordnung und die Ventilanordnung an derselben gesondert vom Behälterkörper ausgebildeten Einsatzanordnung vorgesehen.

Die genannten Einsatzanordnungen, bevorzugt die genannte gemeinsame Einsatzanordnung, kann durch eine am Behälterkörper festlegbare Überwurfanordnung am Behälterkörper gesichert sein. Da ein Laborbehälterhals üblicherweise außen ein Gewinde aufweist, um darauf einen Behälterdeckel aufzuschrauben, kann die Überwurfanordnung gemäß einer vorteilhaften Weiterbildung der vorliegenden Erfindung am Behälterhals gesichert sein, vorzugsweise lösbar gesichert sein, etwa durch Aufschrauben. Die Überwurfanordnung kann dann als Überwurfdeckelkappe ausgebildet sein, etwa in der Gestalt eines Laborbehälterdeckels, wobei die Überwurfdeckelkappe im Gegensatz zu einem gewöhnlichen Verschlussdeckel einen Durchgang für die Fluidleitung aufweisen kann. Die Überwurfdeckelkappe kann wie ein Behälterdeckel auf den Behälterhals auf- und von diesem abgeschraubt werden.

Die Fluidleitung kann jedoch auch einstückig an einem Behälterdeckel ausgebildet sein.

Um für die Gas-Ausgleichsöffnung eine möglichst große Öffnungsfläche bereitzustellen, kann vorgesehen sein, dass die Gas-Ausgleichsöffnung die Fluidleitung umgibt. Vorzugsweise kann die Gas-Ausgleichsöffnung die Fluidleitung vollständig umgeben.

Die Ringnut und die Fluidleitung sind vorzugsweise konzentrisch vorgesehen, so dass zum einen für die Ringnut eine möglichst große Fläche bereitsteht und sodass zum anderen eine mit dem Laborbehälter arbeitende Person anhand der von außen üblicherweise deutlich erkennbaren Fluidleitung sofort erkennen kann, wo sich die Gas-Ausgleichsöffnung in Form der Ringnut befindet.

Damit die Orientierung der Behälteröffnung, insbesondere der Behälteröffnung am freien Ende eines Behälterhalses, für die Arbeit mit dem Laborbehälter keine Rolle spielt, sind die Ringnut und die Fluidleitung vorzugsweise kollinear zu einer die Behälteröffnung zentral durchsetzenden virtuellen Öffnungsachse angeordnet. Bevorzugt ist die Öffnungsachse die oben genannte Halsachse, wenn der Laborbehälter einen Behälterhals mit der Behälteröffnung aufweist. Der Behälterhals ist wenigstens an seiner Innenwandung in der Regel zylindrisch ausgebildet, so dass die Zylinderachse des zylindrischen Behälterhalses bevorzugt die Halsachse ist.

Konstruktiv kann die Gas-Ausgleichsöffnung als eine die Fluidleitung umgebende, vorzugsweise vollständig umgebende, Ringnut auf einer von dem Behälterkörper wegweisenden Außenfläche der Einsatzanordnung ausgebildet sein. Bevorzugt ist dies eine Stirnfläche der Einsatzanordnung, die eben ausgebildet sein kann und daher das Ausbilden der Ringnut erleichtert.

Damit die Gas-Ausgleichsleitung einfach mit der ringnutförmigen Gas-Ausgleichsöffnung der Einsatzanordnung verbunden werden kann, ist vorteilhafterweise die Außenfläche der Einsatzanordnung im Bereich der Gas-Ausgleichsleitung längs der Kanalverlaufsachse durchbrochen, weist also hier eine längs der Kanalverlaufsachse verlaufende Durchgangsöffnung auf.

Ein Teil der Gas-Ausgleichsleitung kann gesondert von der Einsatzanordnung ausgebildet sein und durch Montieren mit dieser verbunden werden. Dies ermöglicht beispielsweise das Austauschen von Gas-Ausgleichsleitungen an einem Laborbehälter oder ermöglicht die Auswahl einer hinsichtlich Gestalt oder/und Material besonders geeigneten Gas-Ausgleichsleitung aus einer Mehrzahl möglicher Ausgleichsleitungen.

Zur Vermeidung eines unerwünschten Flüssigkeitsdurchgangs durch die Gas-Ausgleichsleitung ist vorzugsweise zwischen der Einsatzanordnung und der Überwurfanordnung eine gasdurchlässige, jedoch flüssigkeitsundurchlässige Membran vorgesehen. In diesem Falle kann die Überwurfanordnung die Membran ohne weitere Befestigungsmittel an der Einsatzanordnung am gewünschten Einsatzort halten.

Zur korrekten Positionierung der Einsatzanordnung an der Behälteröffnung kann die Einsatzanordnung einen radial abstehenden Anschlag aufweisen, welcher zur Anlage an einen Rand der Behälteröffnung ausgebildet ist. Vorzugsweise ist der Anschlag ein umlaufender Ringanschlag. Dies muss jedoch nicht so sein. Stattdessen können auch um die Einsatzöffnung herum eine Mehrzahl von einzelnen Anschlägen ausgebildet sein. Vorzugsweise ist wiederum der Rand der Behälteröffnung der Rand eines freien Endes eines vom übrigen Behälterkörper abstehenden Behälterhalses.

Die vorliegende Erfindung wird nachfolgend anhand der beiliegenden Zeichnungen näher erläutert werden. Es stellt dar:
- Figur 1: eine Schnittansicht durch einen erfindungsgemäßen Laborbehälter in Form eines Zellkulturbehälters, welcher an ein Leitungssystem zur Einleitung von Fluid in den Behälter und zur Ausleitung aus diesem angekoppelt ist und
- Figur 2: eine Schnittansicht des Laborbehälters von Figur 1 in einer weiteren Schwenkstellung.

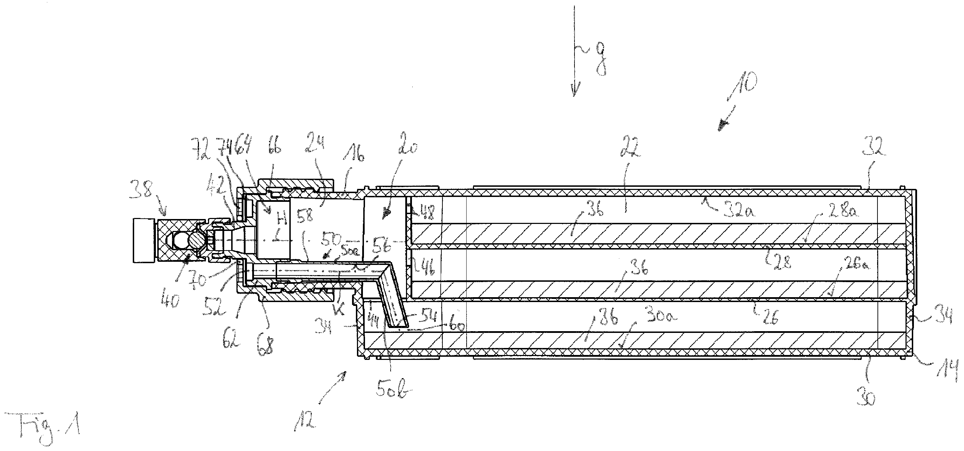

In Figur 1 ist ein erfindungsgemäßer Laborbehälter allgemein mit 10 bezeichnet. Der Laborbehälter weist einen Behälterkörper 12 auf, der einen Behälterhauptkörper 14 und einen davon längs einer Halsachse H abstehenden Behälterhals 16 aufweist. Das vom Laborbehälter 10 umgebene Behältervolumen 20 umfasst daher ein Behälterhauptvolumen 22, welches im Wesentlichen vom Behälterhauptkörper 14 umschlossen ist, und ein vom Behälterhals 16 definiertes Halsvolumen 24.

Im Behälterhauptvolumen 22 können mehrere Zwischenwände 26 und 28 vorgesehen sein, um die für eine Zellkultivierung vorhandene Fläche zu vergrößern. Der Zellkulturbehälter 10 des dargestellten Beispiels dient der Kultivierung von adhärenten Zellen, welche an Innenwänden des Zellkulturbehälters 10 anhaften.

Der Zellkulturbehälter 10, genauer dessen Behälterhauptkörper 14, weist zwei vorzugsweise im Wesentlichen parallele Stirnwandungen 30 und 32 auf, welche durch eine zwischen ihnen liegende Mantelwandung 34 verbunden sind. Von den nach innen zum Behältervolumen 20 hin weisenden Innenwandungen 30a und 32a der Stirnwandungen 30 bzw. 32 ist nur die Innenwandung 30a der Stirnwandung 30 zur Anhaftung adhärenter Zellen ausgerüstet. Die entgegengesetzte Innenwandung 32a der Stirnwandung 32 weist eine vergleichbare Ausrüstung nicht auf.

Ebenso ist von den Zwischenwänden 26 und 28 jeweils nur eine Wandseite zur Anhaftung von adhärenten Zellen ausgebildet, das sind im dargestellten Beispiel die zur Stirnwandung 32 hin weisenden Seiten 26a bzw. 28a.

Im dargestellten Beispiel sind alle zur Anhaftung adhärenter Zellen ausgerüsteten Wandungen 30a, 26a und 28a von einer Nährlösung 36 benetzt, welche über ein hier nicht näher beschriebenes Leitungssystem 38 durch eine mit einer Ventilanordnung 40 wahlweise zur Durchströmung freigebbaren oder verschließbaren Fluidleitung 42 in das Behältervolumen 20 eingeleitet wurde. Durch die Fluidleitung 42 ist das Nährmedium 36 - etwa nach dessen Verbrauch - wieder aus dem Behältervolumen 20 ausleitbar.

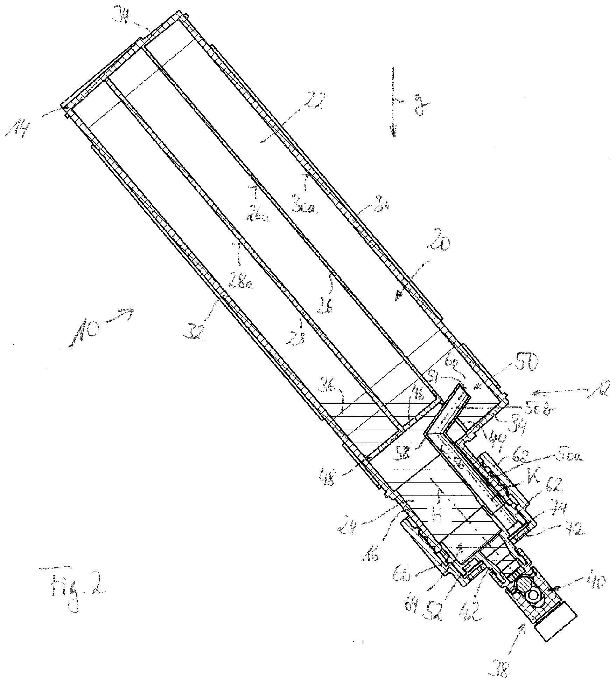

Durch Öffnungen 44, 46 und 48 in dem Zwischenwandsystem kann bei geeigneter Schwenkstellung des Laborbehälters 10 relativ zur Schwerkraftwirkungsrichtung g das Nährmedium 36 von der in Figur 1 dargestellten Benetzungssituation in die in Figur 2 dargestellte fluidleitungsnahe Stellung verbracht werden. Das Nährmedium 36 lässt sich beispielsweise in der in Figur 2 dargestellten Stellung besonders einfach schwerkraftunterstützt aus dem Laborbehälter 10 entnehmen. Hierzu ist es zur restlosen Entleerung des Laborbehälters 10 vorteilhaft, wenn dieser während der Entnahme in eine vertikale Position verbracht wird, in der die Fluidleitung 42 in Schwerkraftwirkungsrichtung g verläuft.

Zum Gasaustausch, insbesondere während einer Einleitung oder Entnahme eines Fluids in das Behältervolumen 20 bzw. aus diesem heraus - jedoch auch für die Dauer der ruhenden Aufbewahrung mit im Behältervolumen 20 vorhandenem Nährmedium 36 - ist am Laborbehälter 10 eine Gas-Ausgleichsleitung 50 vorgesehen, welche sich von einer Gas-Ausgleichsöffnung 52 bis zu einer im Behältervolumen 20, genauer im Behälterhauptvolumen 22, gelegenen Öffnung 54 erstreckt.

Die Gas-Ausgleichsleitung 50 erstreckt sich längs einer Kanalverlaufsachse K und ist von einer Kanalwandung 56 radial nach außen begrenzt.

Ein zur Gas-Ausgleichsleitung 50 beitragendes Rohr 58 oder allgemein die Gas-Ausgleichsleitung 50 kann ein- oder mehrfach abgewinkelt oder gekrümmt sein, um die Öffnung 54 näher an eine Innenwandung heranzuführen. Im dargestellten Ausführungsbeispiel ist dies die zur Anhaftung adhärenter Zellen ausgerüstete Innenwandung 30a der Stirnwandung 30. Zu dieser Innenwandung 30a ist der Rand der Öffnung 54 vorzugsweise parallel angeordnet, so dass zwischen der Innenwandung 30a und der Öffnung 54 ein Ringspalt 60 besteht, welcher eine konstante Höhe aufweist.

Die Gas-Ausgleichsleitung 50 kann, muss jedoch keinen kreisförmigen Querschnitt aufweisen. Vielmehr kann beispielsweise die zur Kanalverlaufsachse K orthogonale Abmessung in Richtung orthogonal zur Zeichenebene der Figur 1 größer sein als die in der Zeichenebene der Figur 1 liegende zur Kanalverlaufsachse K orthogonale Abmessung.

Zur einfachen Montage, insbesondere auch zur Nachrüstung bestehender Laborbehälter 10 kann die Gas-Ausgleichsöffnung 52 an einer Einsatzanordnung 62 ausgebildet sein, welche axial längs der Halsachse H von der Behälteröffnung 64 her in den Behälterhals 16 eingesetzt sein kann.

Um eine möglichst große Öffnungsfläche für die Gas-Ausgleichsöffnung 52 bereitstellen zu können, ist die Gas-Ausgleichsöffnung 52 vorteilhaft ringförmig um die Fluidleitung 42 herum verlaufend ausgebildet. Bevorzugt sind die ringförmige Gas-Ausgleichsöffnung 52 und die Fluidleitung 42 koaxial mit der Halsachse H als gemeinsamer Achse angeordnet.

Zur Erleichterung der Fertigung und Montage ist auch die Fluidleitung 42 bevorzugt an der Einsatzanordnung 62 ausgebildet. Die Einsatzanordnung 62 kann beispielsweise durch Spritzgießen aus Kunststoff gebildet sein. Die Ventilanordnung 40 kann auf die Fluidleitung 42 aufgesteckt, insbesondere aufgeklipst, und verrastet sein.

Zur Lagesicherung der Einsatzanordnung 62 am Behälterhals 16 weist das Einsatzbauteil vorteilhafterweise einen nach außen vorspringenden Radialvorsprung 66 auf, welcher im fertig montierten Zustand am Rand des Behälterhalses 16 anliegt.

Die Einsatzanordnung 62 kann dabei vorteilhafterweise durch eine Überwurfdeckelkappe 68 dauerhaft, aber vorzugsweise lösbar am Behälterhals 16 fixiert sein.

Die Überwurfdeckelkappe 68 kann hierzu in an sich bekannter Weise wie ein ansonsten die Behälteröffnung 64 verschließender Behälterverschlussdeckel mittels eines an ihr ausgebildeten Innengewindes an das am Behälterhals 16 ausgebildete Außengewinde aufgeschraubt sein. Im Gegensatz zu einem herkömmlichen Behälterverschlussdeckel weist die Überwurfdeckelkappe 68 eine von der Fluidleitung 42 durchsetzte Durchsetzungsöffnung 70 auf.

Der in Figur 1 gezeigte Laborbehälter 10 kann nach Abnahme der Überwurfdeckelkappe 68 und nach Entfernen der Einsatzanordnung 62 mit der Gas-Ausgleichsleitung 50 durch einen herkömmlichen Behälterdeckel wie ein herkömmlicher Laborbehälter verschlossen werden.

Die Überwurfdeckelkappe 68 weist außerdem eine Mehrzahl von Belüftungslöchern 72 auf, um zu ermöglichen, dass Luft aus der Außenumgebung des Laborbehälters 10 zur Gas-Ausgleichsöffnung 52 gelangt. Um einen Flüssigkeitsdurchtritt von den Belüftungslöchern 72 in das Behältervolumen 20 hinein oder aus dem Behältervolumen 20 hinaus in die Außenumgebung des Laborbehälters 10 zu verhindern, ist vorteilhafterweise zwischen der die Gas-Ausgleichsöffnung 52 aufweisenden Seite der Einsatzanordnung 62 und der dieser gegenüber liegenden, die Belüftungslöcher 72 aufweisenden Seite der Überwurfdeckelkappe 68 eine gasdurchlässige, aber flüssigkeitsundurchlässige Membran 74 angeordnet.

Die Gas-Ausgleichsleitung 50 kann einstückig oder kann mehrteilig ausgebildet sein. In dem in den Figuren 1 und 2 dargestellten Beispiel ist die Gas-Ausgleichsleitung 50 zweiteilig ausgebildet, mit einem ersten, unmittelbar an die Gas-Ausgleichsöffnung 52 anschließenden Leitungsstück, welches in der Einsatzanordnung 62 ausgebildet ist, und mit dem Rohrstück 58.

Die Gas-Ausgleichsleitung 50 kann einen im Behälterhals 16 verlaufenden Halsleitungsabschnitt 50a und einen im Behälterhauptvolumen 22 verlaufenden Hauptvolumenleitungsabschnitt 50b aufweisen.

Die Darstellung von Figur 2 zeigt, wie die bis in das Behälterhauptvolumen 22 hinein reichende Gas-Ausgleichsleitung 50 ein Entleeren oder auch ein Befüllen des Laborbehälters 10 erleichtert. Durch die in das Behälterhauptvolumen 22 hineingeführte Gas-Ausgleichsleitung 50 kann während des Einleitens von Fluid in den Behälter 10 hinein ein sich durch Verringerung des Gasvolumens ohne Verringerung der Gasmenge ansonsten einstellender Überdruck vermieden werden, indem ein Teil des im Behälter 10 vorhandenen Gases ungestört durch die Gas-Ausgleichsleitung 50 entweichen kann.

Ebenso kann Gas bei einer Entnahme von Flüssigkeit aus dem Laborbehälter 10 ohne Weiteres durch die erfindungsgemäße Gas-Ausgleichsleitung 50 in das Behältervolumen 20 einströmen und so einen ausleitungshemmenden Unterdruck im Behältervolumen 20 vermeiden.

Dies gestattet insbesondere die in Figur 2 angedeutete schwerkraftunterstützte Entnahme von Fluiden, insbesondere Flüssigkeiten, aus dem Behältervolumen 20, bei welcher ansonsten die Gas-Ausgleichsöffnung von dem zu entnehmenden Fluid bedeckt wäre und allein die Membran 74 einen Flüssigkeitsdurchtritt vermeiden würde.

## Patentansprüche

1. Laborbehälter (10), insbesondere Zellkulturbehälter, mit einem von einem Behälterkörper (12) umgebenen Behältervolumen (20), das durch eine Behälteröffnung (64) des Behälterkörpers (12) von außen zugänglich ist, wobei der Laborbehälter (10) eine mit dem Behältervolumen (20) kommunizierende Fluidleitung (42) mit einer Ventilanordnung (40) aufweist, wobei die Fluidleitung (42) zum Durchleiten von Fluid (36) ausgebildet ist, um Fluid (36) aus dem Behältervolumen (20) auszuleiten oder/und von außen in dieses einzuleiten, wobei die Fluidleitung (42) durch die Ventilanordnung (40) wahlweise zur Fluiddurchleitung freigebbar oder sperrbar ist, und wobei der Laborbehälter (10) weiter eine Gas-Ausgleichsöffnung (52) aufweist, durch welche Gas im Gegenstrom zu einem etwaigen Fluidstrom in der Fluidleitung (42) von außen in das Behältervolumen (20) einleitbar oder aus diesem ausleitbar ist, wobei die Gas-Ausgleichsöffnung (52) an einer Gas-Ausgleichsleitung (50) vorgesehen ist, welche sich längs einer virtuellen Kanalverlaufsachse (K) erstreckt und einen von einer Kanalwandung (56) umgebenen Leitungskanal aufweist, der ausgehend von der Gas-Ausgleichsöffnung (52) in das Behältervolumen (20) verläuft und in dieses hinein öffnet, und wobei die Behälteröffnung (64) an einem freien, dem Behälterkörper (12) fernen Längsende eines vom übrigen Behälterkörper (12) abstehenden Behälterhalses (16) ausgebildet ist, wobei die Gas-Ausgleichsleitung (50) den Behälterhals (16) durchsetzend in ein an den Behälterhals (16) anschließendes Behälterhauptvolumen (22) hinein reicht.

2. Laborbehälter nach Anspruch 1,
**dadurch gekennzeichnet, dass** eine im Behältervolumen (20) gelegene Öffnung der Gas-Ausgleichsleitung (50) einer der Innenwandungen (30a, 32a) des Behälterkörpers (12) nächstgelegen ist.

3. Laborbehälter nach Anspruch 1 oder 2,
**dadurch gekennzeichnet, dass** er als Wandungen (30, 32, 34) zwei im Wesentlichen parallele Stirnwandungen (30, 32) und eine diese verbindende Mantelwandung (34) aufweist, wobei die im Behältervolumen (20) gelegene Öffnung (54) der Gas-Ausgleichsleitung (50) einer der Stirnwandungen (30) des Behälterkörpers (12) näher gelegen ist als der jeweils anderen Stirnwandung (32).

4. Laborbehälter nach Anspruch 3,
**dadurch gekennzeichnet, dass** die im Behältervolumen (20) gelegene Öffnung (54) der Gas-Ausgleichsleitung (50) zu der ihr näher gelegenen Stirnwandung (30) hinweist, vorzugsweise dass ein Rand der Öffnung (54) parallel zur Stirnwandung (30) gelegen ist, besonders bevorzugt unter Bildung eines Ringspalts (60) zwischen der Stirnwandung (30) und einem die Öffnung (54) aufweisenden Längsende der Gas-Ausgleichsleitung (50).

5. Laborbehälter nach einem der Ansprüche 3 oder 4,
**dadurch gekennzeichnet, dass** er ein Zellkulturbehälter (10) ist, von dessen Stirnwandungen (30, 32) nur eine Stirnwandung (30) auf ihrer dem Behältervolumen (20) zugewandten Seite (30a) zur Anlage von adhärenten Zellen ausgebildet ist, wohingegen die jeweils andere Stirnwandung (32) auf ihrer dem Behältervolumen (20) zugewandten Seite (32a) eine derartige Ausbildung nicht aufweist, wobei die im Behältervolumen (20) gelegene Öffnung (54) der Gas-Ausgleichsleitung (50) der zur Anlage von adhärenten Zellen ausgebildeten Stirnwandung (30) näher gelegen ist als der jeweils anderen Stirnwandung (32).

6. Laborbehälter nach einem der vorhergehenden Ansprüche,
**dadurch gekennzeichnet, dass** sich der Behälterhals (16) längs einer virtuellen Halsachse (H) erstreckt, dass sich ein im Behälterhals (16) gelegener Halsleitungsabschnitt (50a) der Gas-Ausgleichsleitung (50) parallel zur Halsachse (H) erstreckt, und dass ein in das Behälterhauptvolumen (22) hinein verlaufender, an den Halsleitungsabschnitt (50a) anschließender Hauptvolumenleitungsabschnitt (50b) der Gas-Ausgleichsleitung (50) relativ zum Halsleitungsabschnitt (50b) abgewinkelt oder/und gekrümmt ist.

7. Laborbehälter nach einem der vorhergehenden Ansprüche,
**dadurch gekennzeichnet, dass** die Gas-Ausgleichsöffnung (52) an einer von dem Behälterkörper (12) gesondert ausgebildeten Einsatzanordnung (62) vorgesehen ist, welche in die Behälteröffnung (64) einsetzbar ist.

8. Laborbehälter nach einem der vorhergehenden Ansprüche,
**dadurch gekennzeichnet, dass** die Ventilanordnung (40) an einer von dem Behälterkörper (12) gesondert ausgebildeten Einsatzanordnung (62) vorgesehen ist, welche in die Behälteröffnung (64) einsetzbar ist, vorzugsweise an derselben Einsatzanordnung (62) vorgesehen ist wie die Gas-Ausgleichsöffnung (52).

9. Laborbehälter nach einem der Ansprüche 7 oder 8,
**dadurch gekennzeichnet, dass** die Einsatzanordnung (62) durch eine am Behälterkörper (12), insbesondere am Behälterhals (16), vorzugsweise lösbar, festlegbare Überwurfanordnung (68), insbesondere Überwurfdeckelkappe (68), am Behälterkörper (12) gesichert ist, wobei besonders bevorzugt die Fluidleitung (42) einen Durchgang in der Überwurfanordnung (68) durchsetzt.

10. Laborbehälter nach einem der vorhergehenden Ansprüche,
**dadurch gekennzeichnet, dass** die Gas-Ausgleichsöffnung (52) die Fluidleitung (42) umgibt, vorzugsweise vollständig umgibt.

11. Laborbehälter nach den Ansprüchen 7 und 10,
**dadurch gekennzeichnet, dass** die Gas-Ausgleichsöffnung (52) als eine die Fluidleitung (42) umgebende, vorzugsweise vollständig umgebende Ringnut (52) auf einer von dem Behälterkörper (12) wegweisenden Außenfläche, vorzugsweise Stirnfläche, der Einsatzanordnung (62) ausgebildet ist, wobei besonders bevorzugt die Außenfläche im Bereich der Gas-Ausgleichsleitung (50) längs der Kanalverlaufsachse (K) durchbrochen ist.

12. Laborbehälter nach Anspruch 11,
**dadurch gekennzeichnet, dass** die Ringnut (52) und die Fluidleitung (42) konzentrisch vorgesehen sind, vorzugsweise kollinear zu einer die Behälteröffnung (64) zentral durchsetzenden virtuellen Öffnungsachse.

13. Laborbehälter nach einem der vorhergehenden Ansprüche, unter Einbeziehung des Anspruchs 9,
**dadurch gekennzeichnet, dass** zwischen der Einsatzanordnung (62) und der Überwurfanordnung (68) eine gasdurchlässige, jedoch flüssigkeitsundurchlässige Membran (74) vorgesehen ist.

14. Laborbehälter nach einem der vorhergehenden Ansprüche, unter Einbeziehung des Anspruchs 8 oder 9,
**dadurch gekennzeichnet, dass** die Einsatzanordnung (62) einen radial abstehenden Anschlag (66), vorzugsweise Ringanschlag (66), zur Anlage an einen Rand der Behälteröffnung (64) und damit zur axialen Lagedefinition der Einsatzanordnung (62) relativ zur Behälteröffnung (64) aufweist.

## Claims

1. A laboratory container (10), in particular a cell culture container, with a container volume (20) surrounded by a container body (12) and which is accessible from the outside through a container opening (64) of the container body (12), wherein the laboratory container (10) comprises a fluid line (42) which communicates with the container volume (20) and comprises a valve arrangement (40), wherein the fluid line (42) is constructed for conducting fluid (36) through it in order to conduct fluid (36) out of the container volume (20) and/or from the outside into the latter, wherein the fluid line (42) can be selectively released or blocked by the valve arrangement for conducting fluid through it, and wherein the laboratory container (10) furthermore comprises a gas balancing opening (52) through which gas can be introduced in counterflow to any fluid current in the fluid line (42) from the outside into the container volume (20) or discharged out of the latter,wherein the gas balancing opening (52) is provided on a gas balancing line (50) which extends along a virtual channel course axis (K) and comprises a line channel which is surrounded by a channel wall (56) and which runs starting from the gas balancing opening (52) into the container volume (20) and opens into the latter, and wherein the container opening (64) is formed on a free longitudinal end, remote from the container body (12), of a container neck (16) projecting from the rest of the container body (12), wherein the gas balancing line (50), running through the container neck (16), extends into a main container volume (22) following the container neck (16).

2. The laboratory container according to Claim 1,
**characterized in that** an opening of the gas balancing line (50) located in the container volume (20) is arranged next to one of the inner walls (30a, 32a) of the container body (12).

3. The laboratory container according to Claim 1 or 2,
**characterized in that** it comprises as walls (30, 32, 34) two substantially parallel front walls (30, 32) and a jacket wall (34) connecting them, wherein the opening (54) of the gas balancing line (50) located in the container volume (20) is located closer to one of the front walls (30) of the container body (12) than to the particular other front wall (32).

4. The laboratory container according to Claim 3,
**characterized in that** the opening (54) of the gas balancing line (50) located in the container volume (20) faces the front wall (30) which is closer to it, preferably that an edge of the opening (54) is located parallel to the front wall (30), especially preferably forming an annular slot (50) between the front wall (30) and a longitudinal end of the gas balancing line (50) comprising the opening (54).

5. The laboratory container according to one of Claims 3 or 4,
**characterized in that** it is a cell culture container (10) of whose front walls (30, 32) only one front wall is (30) constructed on its side (30a) facing the container volume (20) for the depositing of adherent cells, in contrast to which the other front wall (32) does not have such a construction on its side (32a) facing the container volume (20), wherein the opening (54) of the gas balancing line (50) located in the container volume (20) is located closer to the front wall (30) constructed for the depositing of adherent cells than it is to the particular other front wall (32).

6. The laboratory container according to one of the previous claims,
**characterized in that** the container neck (16) extends along a virtual neck axis (H), that a neck line section (50a) of the gas balancing line (50) and located in the container neck (16) extends parallel to the neck axis (H), and that a main volume line section (50b) of the gas balancing line (50), which section runs into the main container volume (22) and follows the neck line section (50a) is bent and/or curved relative to the neck line section (50b).

7. The laboratory container according to one of the previous claims,
**characterized in that** the gas balancing opening (52) is provided on an insert arrangement (62) constructed separately from the container body (12) and which can be set into the container opening (64).

8. The laboratory container according to one of the previous claims,
**characterized in that** the valve arrangement (40) is provided on an insert arrangement (62) constructed separately from the container body (12) and which can be set into the container opening (64) and is preferably provided on the same insert arrangement (62) as the gas balancing opening (52).

9. The laboratory container according to one of the previous claims 7 or 8,
**characterized in that** the insert arrangement (62) is secured by a clasp arrangement (68), in particular a clasp cover cap (68) on the container body (12), which clasp arrangement can preferably be detachably fixed on the container body (12), in particular on the container neck (16), wherein the fluid line (42) especially preferably runs through a passage in the clasp arrangement (68).

10. The laboratory container according to one of the previous claims,
**characterized in that** the gas balancing opening (52) surrounds the fluid line (42), preferably completely.

11. The laboratory container according to Claims 7 and 10,
**characterized in that** the gas balancing opening (52) is constructed as an annular groove (52) which surrounds the fluid line (42) preferably completely on an outer surface, preferably a front surface, of the insert arrangement (62) facing away from the container body (12), wherein the outer surface is especially preferably perforated in the area of the gas balancing line (50) along the channel course axis (K).

12. The laboratory container according to Claim 11,
**characterized in that** the annular groove (52) and the fluid line (42) are concentrically provided, preferably colinearly to a virtual opening axis running centrally through the container opening (64).

13. The laboratory container according to one of the previous claims, with the inclusion of Claim 9,
**characterized in that** a gas-permeable but liquid-impermeable membrane (74) is provided between the insert arrangement (62) and the clasp arrangement (68).

14. The laboratory container according to one of the previous claims, with the inclusion of Claim 8 or 9,
**characterized in that** the insert arrangement (62) comprises a radially projecting stop (66), preferably an annular stop (66) for contacting an edge of the container opening (64) and therefore for the axial definition of the position of the insert arrangement (62) relative to the container opening (64).

## Revendications

1. Récipient de laboratoire (10), en particulier récipient de culture cellulaire, avec un volume de récipient (20) entouré par un corps de récipient (12), accessible de l'extérieur par un orifice de récipient (64) du corps de récipient (12), le récipient de laboratoire (10) comprenant une conduite de fluide (42) communiquant avec le volume de récipient (20), avec un arrangement de soupape (40), la conduite de fluide (42) étant adaptée pour faire passer du fluide (36), pour évacuer du fluide (36) du volume de récipient (20) ou/et pour introduire du fluide dans celui-ci de l'extérieur, la conduite de fluide (42) pouvant sélectivement être libérée ou bloquée par l'arrangement de soupape (40) pour le passage de fluide, et le récipient de laboratoire (10) comprenant en outre un orifice de compensation de gaz (52) par laquelle du gaz peut, à contre-courant à un éventuel courant de fluide dans la conduite de fluide (42), être introduit dans ou évacué du volume de récipient (20) de l'extérieur, l'orifice de compensation de gaz (52) étant prévue à une conduite de compensation de gaz (50) qui s'étend le long d'un axe du canal (K) virtuel et comprend un canal de conduite entouré par une paroi de canal (56), qui s'étend de l'orifice de compensation de gaz (52) dans le volume de récipient (20) et s'ouvre dans celui-ci, et l'orifice de récipient (64) étant formée à une extrémité longitudinale libre, éloignée du corps de récipient (12), d'un goulot de récipient (16) mis en avant du reste du corps de récipient (12), la conduite de compensation de gaz (50) passant par le goulot de récipient (16) et s'étendant dans un volume principal de récipient (22) associé au goulot de récipient (16).

2. Récipient de laboratoire selon la revendication 1,
**caractérisé en ce qu'**un orifice de conduite de compensation de gaz (50) située dans le volume de récipient (20) est le plus proche à une des parois intérieures (30a, 32a) du corps de récipient (12).

3. Récipient de laboratoire selon la revendication 1 ou 2,
**caractérisé en ce qu'**il comprend, en tant que parois (30, 32, 34) deux parois frontales (30, 32) essentiellement parallèles et une paroi d'enveloppe (34) liant celles-ci, l'orifice (54) de la conduite de compensation de gaz (50) situé dans le volume de récipient (20) étant plus proche à l'une des parois frontales (30) du corps de récipient (12) qu'à l'autre paroi frontale respective (30).

4. Récipient de laboratoire selon la revendication 3,
**caractérisé en ce que** l'orifice (54) de la conduite de compensation de gaz (50) situé dans le volume de récipient (20) est orienté vers la paroi frontale (30) plus proche de ce dernier, de préférence **en ce qu'**un bord de l'orifice (54) est parallèle à la paroi frontale (30), de manière particulièrement préférée en formant une fente annulaire (60) entre la paroi frontale (30) et une extrémité longitudinale de la conduite de compensation de gaz (50) comprenant l'orifice (54).

5. Récipient de laboratoire selon une des revendications 3 ou 4,
**caractérisé en ce qu'**il s'agit d'un récipient de culture cellulaire (10) des parois frontales (30, 32) duquel seulement une paroi frontale (30) est adaptée sur son côté (30a) en face du volume de récipient (20) pour l'arrangement de cellules adhérentes, tandis que l'autre paroi frontale respective (32) ne comprend pas une telle structure sur son côté (32a) orienté vers le volume de récipient (20), l'orifice (54) situé dans le volume de récipient (20) de la conduite de compensation de gaz (50) étant plus proche de la paroi frontale (30) adaptée pour l'arrangement de cellules que de l'autre paroi frontale respective (32).

6. Récipient de laboratoire selon une des revendications précédentes,
**caractérisé en ce que** le goulot de récipient (16) s'étend le long d'un axe de goulot virtuel (H), qu'une section de conduite de goulot (50a) située dans le goulot de récipient (16) de la conduite de compensation de gaz (50) s'étend parallèlement à l'axe de goulot (H) et **en ce qu'**une section de conduite de volume principal (50b) de la conduite de compensation de gaz (50) qui s'étend dans le volume principal de récipient (22) et qui est associée à la section de conduite de goulot (50a) est inclinée ou/et courbée par rapport à la section de conduite de goulot (50b).

7. Récipient de laboratoire selon une des revendications précédentes,
**caractérisé en ce que** l'orifice de compensation de gaz (52) est prévu à un arrangement d'insertion (62) formé séparément du corps de récipient (12) qui peut être inséré dans l'orifice de récipient (64).

8. Récipient de laboratoire selon une des revendications précédentes,
**caractérisé en ce que** l'arrangement de soupape (40) est prévu à un arrangement d'insertion (62) formé séparément du corps de récipient (12) qui peut être inséré dans l'orifice de récipient (64), de préférence au même arrangement d'insertion (62) que l'orifice de compensation de gaz (52).

9. Récipient de laboratoire selon une des revendications 7 ou 8,
**caractérisé en ce que** l'arrangement d'insertion (62) est sécurisé au corps de récipient (12) par un arrangement de couverture (68), en particulier un capuchon de couvercle de couverture (68) qui peut être fixé au corps de récipient (12), de préférence au goulot de récipient (16), de préférence de manière amovible, où de manière particulièrement préférée la conduite de fluide (42) passe à travers un passage dans l'arrangement de couverture (68).

10. Récipient de laboratoire selon une des revendications précédentes,
**caractérisé en ce que** l'orifice de compensation de gaz (52) renferme la conduite de fluide (42), de préférence entièrement.

11. Récipient de laboratoire selon les revendications 7 et 10,
**caractérisé en ce que** l'orifice de compensation de gaz (52) est adapté en tant que rainure annulaire (52) entourant la conduite de fluide (42), de préférence entièrement, sur une surface extérieure loin du corps de récipient (12), de préférence surface frontale, de l'arrangement d'insertion (62), la surface extérieure étant de manière particulièrement préférée interrompue dans la région de la conduite de compensation de gaz (50) le long de l'axe du canal virtuel (K).

12. Récipient de laboratoire selon la revendication 11,
**caractérisé en ce que** la rainure annulaire (52) et la conduite de fluide (42) sont prévues de manière concentrique, de préférence de manière colinéaire à un axe d'orifice virtuel passant centralement à travers l'orifice de récipient (64).

13. Récipient de laboratoire selon une des revendications précédentes, en considérant la revendication 9,
**caractérisé en ce qu'**une membrane (74) perméable au gaz et nonperméable au fluide, est prévue entre l'arrangement d'insertion (62) et l'arrangement de couverture (68).

14. Récipient de laboratoire selon une des revendications précédentes, en considérant la revendication 8 ou 9,
**caractérisé en ce que l**'arrangement d'insertion (62) comprend un arrêt (66) en saillie radiale, de préférence un arrêt annulaire (66) pour l'appui contre un bord de l'ouverture de récipient (64) et ainsi pour la définition de situation axiale de l'arrangement d'insertion (62) par rapport à l'orifice de récipient (64).
